# EUROPEAN PATENT APPLICATION

(11) **EP 2 702 991 A1**
(43) Date of publication of application: **05.03.2014**
(21) Application number: 11864150.5
(22) Date of filing: 26.04.2011
(51) Int. Cl.: A61K 31/275, A61K 47/32, A61K 47/02, A61P 25/16

(54) **A COMPOSITION OF ENTACOPONE**

(71) Applicant: Innopharmax,inc., Taipei City, Taiwan 11492 (TW)
(72) Inventor: HAO, Wei-Hua, Taipei City Taiwan 11492 (CN); WANG, Jong-Jing, Taipei City Taiwan 11492 (CN); CHEN, Hui-Yun, Taipei City Taiwan 11492 (CN)
(74) Representative: Becker Kurig Straus
(86) International application number: PCT/CN2011/073297
(87) International publication number: WO 2012/145893

(57) **Abstract**

A composition of entacapone comprising entacapone or pharmaceutically acceptable salts, PVPK30 and SDS is disclosed in the present invention, wherein the mass ratio of entacapone : PVPK30 : SDS is 1:0.05~0.6:0.06~0.1. The present invention also discloses preparative method and use of the composition of entacapone.

## Description

### FIELD OF THE INVENTION

The present invention relates to a composition of entacapone, comprising entacapone, PVP K30 and SDS. In addition, the present invention also relates to a process for preparing said composition of entacapone and uses thereof.

### BACKGROUND OF THE INVENTION

Entacapone, also known as (E)-2-cyano-3-(3,4-dihydroxy-5-nitro-phenyl) -N,N-diethyl-2-propenamide, is a medicine presently known for treating Parkinson's Disease.

Since entacapone has a low dissolution rate and low bio-availability, presently known compositions of entacapone are mostly made to be smaller-sized granules by grinding or crushing so as to increase its dissolution rate and bio-availability. For example, US 2010/0104634 A1 made 90% of entacapone granules have a particle size of less than 40 µm, while WO 2009098661 A1 made granules of entacapone and sugar alcohols go through a micronization process so that the particle size is less than 30 µm.

However, techniques such as grinding or micronization usually generate high heat and thus make the drugs deteriorate. In addition, these techniques still have problems like low productivity and high costs. Therefore, a pressing problem to be solved in the art is how to prepare a composition of entacapone with a high dissolution rate or high bio-availability without utilizing techniques like grinding, crushing or micronization.

### BRIEF SUMMARY OF THE INVENTION

To solve the aforementioned problems, the inventors have developed an improved process which can increase the dissolution rate of entacapone without utilizing techniques like grinding, crushing or micronization, and also increase productivity and reduce production costs by simplifying the process.

One of the objectives of the present invention is to provide a composition of entacapone, comprising entacapone or a pharmaceutically acceptable salt thereof, PVP K30 (polyvinylpyrrolidone K30), SDS (sodium dodecyl sulfate), and at least one pharmaceutically acceptable excipient.

To achieve the above objective, the present invention provides a composition of entacapone comprising entacapone or a pharmaceutically acceptable salt thereof, PVP K30 and SDS; wherein entacapone, PVP K30 and SDS are present at a weight ratio of 1:0.05-0.6:0.06-0.1, and the composition has a dissolution rate of more than 88%.

In a preferred embodiment of the present invention, in said composition of entacapone, entacapone, PVP K30 and SDS are present at a weight ratio of 1:0.05-0.2:0.06; more preferably, 1:0.2:0.06.

In a preferred embodiment of the present invention, said composition of entacapone has a dissolution rate of more than 90%; more preferably, the dissolution rate is more than 95%.

In a preferred embodiment of the present invention, said composition of entacapone further comprises at least one excipient, and said excipient is selected from povidone, crospovidone, carbohydrates, croscarmellose sodium, or combinations thereof. Said carbohydrate can be selected from, for example, microcrystalline cellulose (MCC), mannitol, cellulose, hydroxypropyl methylcellulose, starch, or lactose.

The present invention also provides a process for preparing the composition of entacapone as described above, comprising the following steps:
(a) mixing entacapone or a pharmaceutically acceptable salt thereof, PVP K30 and SDS, wherein entacapone, PVP K30 and SDS are present at a weight ratio of 1:0.05-0.6: 0.06-0.1;
(b) sieving the mixture obtained in step (a) through a sieve, wherein the sieve has a mesh size smaller than 180 µm, more preferably smaller than 150 µm;
(c) granulating the mixture obtained in step (b) to obtain granules;
(d) drying the granules obtained in step (c) until the water content thereof is between 1% and 3%, obtaining said composition of entacapone;
the process does not comprise any step of grinding, crushing or micronizing the mixture of entacapone or a pharmaceutically acceptable salt thereof, PVP K30 and SDS to make the particle size of the mixture less than 30 µm.

In a preferred embodiment of the present invention, in step (a) of said process, entacapone, PVP K30 and SDS are present at a weight ratio of 1:0.05-0.2:0.06; more preferably, 1:0.2:0.06.

In a preferred embodiment of the present invention, in step (c) of said process, the granulating step is wet granulation.

In a preferred embodiment of the present invention, said process does not comprise any step of grinding, crushing or micronizing the mixture of entacapone, PVP K30 and SDS to make the particle size of the mixture less than 40 µm.

In a preferred embodiment of the present invention, step (a) of said process further comprises at least one excipient, and said excipient is selected from povidone, crospovidone, carbohydrates, croscarmellose sodium, or combinations thereof. Said carbohydrate can be selected from, for example, microcrystalline cellulose, mannitol, cellulose, hydroxypropyl methylcellulose, starch, or lactose.

The present invention further provides a pharmaceutical composition, comprising the aforementioned composition of entacapone; preferably a pharmaceutical composition for treating Parkinson's Disease.

In a preferred embodiment of the present invention, said pharmaceutical composition further comprises levodopa and benserazide, or further comprises levodopa and carbidopa.

The present invention further provides use of the aforementioned composition of entacapone for preparing pharmaceutical compositions; preferably, for preparing pharmaceutical compositions for treating Parkinson's Disease.

In a preferred embodiment of the present invention, pharmaceutical compositions in said use further comprise levodopa and benserazide, or further comprise levodopa and carbidopa.

In view of the above, the composition of entacapone provided in the present invention can increase the dissolution rate of entacapone to more than 88% without utilizing techniques like grinding, crushing or micronization. Compared to known processes, the present invention can not only simplify the process by preventing steps associated with grinding, but also avoid the risk of deterioration of entacapone induced by the high heat resulted from the grinding-associated steps. The present invention can also increase productivity and reduce costs.

### DETAILED DESCRIPTION OF THE INVENTION

The following examples are merely illustrations of the best embodiments but not intended to limit the scope of the present invention. Based on the disclosure of the present invention, those skilled in the art may make appropriate changes and modifications without violating the spirit of the present invention.

### Example

First, granules of entacapone compositions 1 to 6 were prepared according to the ratios of the compositions shown in Table 1 below.

MCC, SDS, PVP K30, mannitol, and entacapone were added sequentially and mixed for 5 minutes. Then, the mixture was sieved through a sieve of 150 µm mesh size (100 mesh). The sieved powder was put into a granulator, sprayed with deionized water and granulated. The resulted granules were placed in a drying oven at 50°C until the water content thereof is between 1% and 3%, and then packed into capsules or pressed into tablets to obtain entacapone compositions 1 to 6.

**Table 1**

| | entacapone (mg) | PVP K30 (mg) | SDS (mg) | MCC (mg) | mannitol (mg) |
|---|---|---|---|---|---|
| Composition 1 | 200 | 0 | 0 | 0.5 | 168 |
| Composition 2 | 200 | 10 | 12 | 0.5 | 168 |
| Composition 3 | 200 | 8 | 8 | 0.5 | 168 |
| Composition 4 | 200 | 20 | 12 | 0.5 | 168 |
| Composition 5 | 200 | 40 | 12 | 0.5 | 168 |
| Composition 6 | 200 | 120 | 20 | 0.5 | 168 |

The dissolution test was conducted according to the drug dissolution method for entacapone obtained by searching the Dissolution Methods Database provided on the FDA website (http://www.accessdata.fda.gov/scripts/cder/dissolution/dsp_Search Results_Dissolutions.cfm). The dissolution test was carried out using a USP standard paddle stirring apparatus with a rotation speed of 50 rpm. The solvent used was 900 mL pH 5.5 phosphate buffer solution and the period of test was 120 minutes. Then the resulting solution was analyzed by HPLC and the obtained dissolution rates were shown in Table 2 below.

**Table 2**

| | entacapone compositions | | | Dissolution Rate (%) |
|---|---|---|---|---|
| | entacapone (mg) | PVP K30 (mg) | SDS (mg) | |
| Composition 1 | 200 | 0 | 0 | 81.15 |
| Composition 2 | 200 | 10 | 12 | 91.78 |
| Composition 3 | 200 | 8 | 8 | 83.20 |
| Composition 4 | 200 | 20 | 12 | 93.72 |
| Composition 5 | 200 | 40 | 12 | 95.98 |
| Composition 6 | 200 | 120 | 20 | 88.93 |

In view of the above, the dissolution rate of entacapone per se was about 81.15%. When the weight ratio of entacapone, PVP K30 and SDS was 1:0.05-0.6:0.06-0.1, the dissolution rate of the resulted entacapone composition increased significantly to reach more than 88%. When the weight ratio of entacapone, PVP K30 and SDS was 1:0.05-0.2:0.06, the dissolution rate of the resulted entacapone composition could reach even more than 90%. Lastly, when the weight ratio of entacapone, PVP K30 and SDS was 1:0.2:0.06, the dissolution rate could reach more than 95%.

Therefore, the entacapone compositions obtained by adopting the aforementioned ratios without utilizing techniques like grinding, crushing or micronization can provide not only advantages such as high dissolution rates and simplified processes, but also avoid the concerns of deterioration of entacapone resulted from the high heat generated during grinding.

## Claims

1. A composition of entacapone, comprising entacapone or a pharmaceutically acceptable salt thereof, PVP K30 and SDS; wherein entacapone, PVP K30 and SDS are present at a weight ratio of 1:0.05-0.6:0.06-0.1, and the composition has a dissolution rate of more than 88%.

2. The composition of entacapone according to claim 1, wherein entacapone, PVP K30 and SDS are present at a weight of 1:0.05-0.2:0.06.

3. The composition of entacapone according to claim 2, wherein entacapone, PVP K30 and SDS are present at a weight of 1:0.2:0.06.

4. The composition of entacapone according to claim 1, wherein the dissolution rate is more than 90%.

5. The composition of entacapone according to claim 4, wherein the dissolution rate is more than 95%.

6. The composition of entacapone according to claim 1, further comprising at least one excipient, and said excipient is selected from povidone, crospovidone, carbohydrates, croscarmellose sodium, or combinations thereof.

7. The composition of entacapone according to claim 6, wherein the carbohydrates are selected from microcrystalline cellulose, mannitol, cellulose, hydroxypropyl methylcellulose, starch, or lactose.

8. A process for preparing the composition of entacapone according to claim 1, comprising the following steps:
(a) mixing entacapone or a pharmaceutically acceptable salt thereof, PVP K30 and SDS, wherein entacapone, PVP K30 and SDS are present at a weight ratio of 1:0.05-0.6: 0.06-0.1;
(b) sieving the mixture obtained in step (a) through a sieve, wherein the sieve has a mesh size smaller than 180 µm;
(c) granulating the mixture obtained in step (b) to obtain granules;
(d) drying the granules obtained in step (c) until the water content thereof is between 1% and 3%, obtaining the composition of entacapone according to claim 1;
the process does not comprise any step of grinding, crushing or micronizing the mixture of entacapone or a pharmaceutically acceptable salt thereof, PVP K30 and SDS to make the particle size of the mixture less than 30 µm.

9. The process according to claim 8, wherein in step (a) entacapone, PVP K30 and SDS are present at a weight ratio of 1:0.05-0.2:0.06.

10. The process according to claim 9, wherein in step (a) entacapone, PVP K30 and SDS are present at a weight ratio of 1:0.2:0.06.

11. The process according to claim 8, wherein in step (c) the granulating step is wet granulation.

12. The process according to claim 8, which does not comprise any step of grinding, crushing or micronizing the mixture of entacapone or a pharmaceutically acceptable salt thereof, PVP K30 and SDS to make the particle size of the mixture less than 40 µm.

13. The process according to claim 8, wherein step (a) further comprises adding at least one excipient, and said excipient is selected from povidone, crospovidone, carbohydrates, croscarmellose sodium, or combinations thereof.

14. The process according to claim 13, wherein the carbohydrates are selected from microcrystalline cellulose, mannitol, cellulose, hydroxypropyl methylcellulose, starch, or lactose.

15. A pharmaceutical composition, comprising the composition of entacapone according to claim 1.

16. The pharmaceutical composition according to claim 15, which is a pharmaceutical composition for treating Parkinson's Disease.

17. The pharmaceutical composition according to claim 16, further comprising levodopa and benserazide, or further comprising levodopa and carbidopa.

18. Use of the composition of entacapone according to claim 1 for manufacturing a pharmaceutical composition.

19. The use according to claim 18, wherein said pharmaceutical composition is one for treating Parkinson's Disease.

20. The use according to claim 18, wherein said pharmaceutical composition further comprises levodopa and benserazide, or further comprising levodopa and carbidopa.
